# EUROPEAN PATENT SPECIFICATION

(11) **EP 3 205 308 B1**
(45) Date of publication and mention of the grant of the patent: **02.04.2025**
(21) Application number: 16155282.3
(22) Date of filing: 11.02.2016
(51) Int. Cl.: A61F 2/24

(54) **STENT**
STENT
ENDOPROTHESE

(43) Date of publication of application: 16.08.2017
(73) Proprietor: P+F Products + Features GmbH, 1190 Wien (AT)
(72) Inventor: KISS, Katharina, 1190 Wien (AT)
(74) Representative: Manitz Finsterwald Patent- und Rechtsanwaltspartnerschaft mbB

(56) References cited:
- US-A1- 2007 239 273
- US-A1- 2008 046 071
- US-A1- 2014 277 389
- US-A1- 2014 277 563
- US-A1- 2015 216 658

## Description

The present invention relates to a stent for a surgical valve for placement at the aortic annulus that is expandable from an un-deployed state into a deployed state.

A healthy heart facilitates oxygenated blood flow to the extremities. The heart is comprised of two chambers: the right and left, which manage deoxygenated and oxygenated blood respectively. Deoxygenated blood from the upper and the lower extremities, travels through the caval veins into the right atrium. It is pumped through the tricuspid valve and into the right ventricle. During systole, blood is pumped from the right ventricle, through the pulmonary valve and into the lungs.

Following oxygenation, blood is pumped back to the left side of the heart through the pulmonary artery. Oxygenated blood flows through the mitral valve into the left ventricle. During systole, the left ventricle ejects the blood through the aortic valve to the rest of the body.

A native aortic valve consists of three leaflets that are attached directly to the wall of the annulus. There are three cusps in the native aortic valve: the left coronary cusp (LCC), right coronary cusp (RCC), and the non-coronary cusp (NCC). The bellies of the cusps define the annulus of the native aortic valve. The annulus is predominantly circular or elliptical in shape. At the location of the cusp attachment, the aortic anatomy is typically at its largest diameter, and this defines the sinus region. The sinus of the aortic anatomy is critical to the healthy functioning of the heart as the coronary arteries, which carry oxygenated blood to the heart muscles, originate here.

In patients with aortic stenosis, the integrity of the native leaflets and the annulus is compromised primarily due to calcification. A compromised valvular apparatus prevents the leaflets from fully opening during systole, thus affecting the hemodynamic functioning of the valve. In instances where this condition persists for an extended period of time, the left ventricle remodels to compensate, affecting cardiac performance and patient health.

Surgical aortic valve replacement offers physicians the greatest flexibility regarding implant type - with choices of either a mechanical or bioprosthetic heart valve. The decision of whether an operation is possible is based on many factors, including age, surgeon preference, patient tolerance to blood thinners, and comorbidities. Surgical replacement is recommended for patients who are a low or intermediate surgical risk. Elderly patients who are at a high surgical risk or inoperable due to comorbidities require a different alternative.

One drawback of existing technologies is leakage around the valve, termed paravalvular leakage (PVL). PVL is usually a result of at least one of the following mal-positioning of an implant, calcium interference with implant expansion, incorrect sizing of the implant and/or implant migration.

Prior art stent designs are disclosed in the following documents US2014/277389A1, US2014/277563A1, US2015/216658A1, US2008/046071A1, and US2007/239273A1.

For this reason it is an object of the present invention to make available a stent by means of which PVL is prevented as far as possible. It is a further object to make available a stent that has an increased flexibility in order to be transported to and be placed more easily at the position of deployment. It is a further object of the invention to provide a stent that reduces trauma during surgery, so that the stent can also be used with elderly patients.

This object is satisfied by a stent having the features of claim 1. Specific embodiments of the invention are described in the dependent claims.

Such a stent for placement at the aortic annulus that is expandable from an un-deployed state into a deployed state in particular comprises:
a stent frame that has an inferior section and a superior section and that is formed from a plurality of elongate struts, with the plurality of elongate struts being connected one to another at points of connection to form a plurality of stent cells formed of vertices at the points of connection and respective sections of the plurality of elongate struts; and
a plurality of eyelets, with at least some of the plurality of eyelets forming at least some of the vertices at the points of connection between the elongate struts wherein at least some of the elongate struts are endless struts having an undulating shape with two end points and several eyelets, with the vertices of at least some of the stent cells of the endless struts respectively being formed by points of connection between adjacent elongate struts and by points of constriction by means of which the endless struts are respectively combined along their length to form stent cells within an individual endless strut, and wherein at least some of the stent cells have all of their vertices formed by the eyelets, with the eyelets connecting respective sections of the elongate struts.

The stent for an implant or prosthesis described herein is in particular suitable for the treatment of aortic stenosis via minimally invasive transcatheter implantation to replace a defective aortic valve.

The use of eyelets to form at least some of the vertices and hence points of connection between individual struts renders the stent more flexible. This is because the eyelets are generally less rigid than points of connection of prior art design.

In this connection it should be noted that the term vertex refers to a corner region of each cell, i.e. the region of the cell forming a corner where two respective sections of the struts meet. Preferably the vertex at least substantially forms an origin of the corner where the two respective sections of the struts meet.

Thus, when an eyelet is used to form the vertex of a cell the eyelet also at least substantially forms the origin of the corner where the two respective sections of the struts meet. Preferably the vertices form corners of the plurality of stent cells.

It should further be noted in this connection that the sections of the plurality of stents forming sides of the stent cells can be of linear, curved or zigzag shape.

In this way stent cells can be formed that advantageously comprise an oval, curved diamond or diamond shape. Such shapes can be manufactured in a simple manner and provide the stent frame with an increased stability and flexibility. Moreover, a curved surface of the stent cells reduces a likelihood of puncturing a blood vessel on contact with the blood vessel.

Due to the increased flexibility of the stent, the implant comprising such a stent can be placed more accurately within a blood vessel thereby improving the function of the implant due to the increased apposition to the native annular anatomy using appropriate design options. Flexibility within the stent is intended to aid implant trackability within the vasculature. In addition, the ability to steer the implant from the access site to the implant site is improved due the increased flexibility even in the un-deployed state of the stent. This is necessary to reduce trauma to a patient during implantation, and to ensure accurate implant placement. The reduction of trauma to a patient also makes the surgery less critical so that this kind of implant can also be provided in patients who were previously not operable due to the too high a risk associated with the implantation of prior art designs.

Preferably the material of the stent frame can be selected from the group of materials comprising a superelastic material, a shape memory material, a binary, ternary or quaternary nitinol. Additional elements may consist of, but are not limited to chromium, tantalum, gold, and others to either enhance the radiopacity of the frame, the mechanical properties of the frame or any combination thereof. It should be noted that the stent frame can be laser cut from a tube of material and processed to form a continuous frame. In this way the stent frame and hence the stent can be manufactured in a simple manner using existing technologies that facilitate the manufacture of the stent frame.

Advantageously at least some of the stent cells consist of vertices formed by some of the plurality of eyelets and of respective sections of the plurality of elongate struts. Forming at least some stent cells having all of their vertices formed by eyelets that connect respective sections of the struts improves stent flexibility, manufacturability, and also aids tracking of the implant for deployment at the site of the native annulus through various transcatheter approaches.

Preferably all of the vertices of stent cells formed in a common plane of the stent frame are formed by some of the plurality of eyelets.

Forming a stent frame in which specific planes or regions of the stent have a greater or lesser flexibility is beneficial as in this way different regions of the stent can be provided with varying flexibility which means the stent can be adapted for specific uses and diverse implantation sites.

It is preferred if at least some of the stent cells only comprise at most three vertices formed by some of the plurality of eyelets. In this way the remaining vertex or vertices may take on a different function. For example, attachment means could be attached at the remaining vertex or vertices. Alternatively the remaining vertex or vertices could be formed by an elongate strut itself or by a different kind of connection.

Advantageously at least some of the elongate struts are endless struts, with the vertices of at least some of the stent cells of the endless struts respectively being formed by points of connection between adjacent elongate struts and by points of constriction by means of which the endless struts are respectively combined along their length to form stent cells within an individual endless strut.

Providing endless struts as struts provides the stent frame with greater stability at its distal and proximal ends respectively and also ensures a more cost-effective manufacture of the stent frame. Moreover, the eyelets used as points of constriction along the length of the endless strut can be positioned at varying spacings along the length of the endless strut to form different size stent cells along the length of each endless strut.

For example, the size of each stent cell can respectively increase along the length of each endless strut from the inferior section to the superior section and vice ver-sa. Alternatively the stent cells formed in a central region of the stent frame can be larger than the respective stent cells arranged at the distal and proximal regions.

Advantageously the plurality of eyelets each form a respective origin of the points of connection and/or of the points of constriction. In this way the eyelets are arranged directly in a corner of each stent cell increasing the stability of the corner region while simultaneously increasing the flexibility of the stent.

Preferably, stent cells of varying size are arranging at different positions along the length of a stent frame. In this way a stent frame can be provided which has different elastic properties in different regions of the stent frame, with the elastic properties preferably being selected in accordance with the respective blood vessel into which the stent is to be introduced.

Advantageously all of the points of constriction and/or all of the points of connection are formed by the plurality of eyelets. In this way the stent frame can be manufactured with a significantly increased flexibility and the advantages associated therewith.

Preferably a respective end of an endless strut does not comprise a point of connection or a point of constriction.

It should be noted in this connection that the eyelets forming at least some of points of connection preferably do not form points of constriction.

It is preferred if the inferior section comprises a larger number of eyelets than the superior section.

Providing a larger number of eyelets in the inferior section compared to the superior section means that the stent cells of the inferior section are smaller. Thus the properties of the inferior section can be selected different from the properties of the superior section, so that each section can be tailored to specific requirements.

Advantageously the inferior section is less flexible than the superior section. It should nevertheless be noted that the inferior section is still more flexible than that of prior art designs. In this way the implant can be designed with greater flexibility while at the same time maintaining or even improving the function of the replacement valve. In this connection it should be noted that an implant comprises a stent as described herein in combination with a surgical valve - also known as a replacement valve or a valvular apparatus.

By designing the superior section more flexible than the inferior section the outer shape of the superior section can adapt more easily to an inner contour of the blood vessel to which the implant is attached. In this way the superior section is also configured for an improved interference fit with the blood vessel into which it is implanted. An improved interference fit also reduces the likelihood of occurrence of paravalvular leakage.

In this connection it is also preferable if the superior section has a larger outer diameter than the inferior section to thereby further improve an interference fit between the stent and the blood vessel.

In this connection it should be noted that a shape of each section (superior and/or inferior) in a plane perpendicular to the longitudinal extent of the stent can be selected as oval, circular or elliptical or any combination thereof.

In this way the geometry of the stent is contoured to allow an improved functioning of the tissue valve at or around the location of the native aortic valve. The implant has a profile tailored to adapt to the geometry of the aortic anatomy. Specifically, the implant has a rounded contour with curved diamond cells within the annular area for valve function.

Preferably at least one, in particular at least two, of the plurality of eyelets forming one of the vertices is provided with a radiopaque marker (RO). The RO marker is indicative of the positioning of the valve, is an aid for the deployment of the valve during a procedure, and enables the monitoring of the functioning of the valve with respect to defined markers.

In this connection it should be noted that the material of the eyelets may include gold, tantalum, platinum, a combination of any of the above, and other biocompatible materials.

In a preferred embodiment the stent further comprises a cuff material, with the cuff material being arranged to cover at least part of an inner and/or an outer surface of the stent frame and/or with the cuff material being arranged to at least substantially completely cover at least the inferior section and/or the superior section.

The cuff material may be attached to the stent frame by at least one of the following ways including sutures, adhesively bonding, welding, brazing, clamps, barbs, hooks and other miscellaneous methods of attachment. Attachment of the cuff may be directly to or around the stent struts or through the eyelets.

The use of a cuff material also mitigates paravalvular leakage this in combination with an improved interference fit with the anatomy by means of a specific shaping of the superior section significantly improves the design of the stent and hence even further mitigates paravalvular leakage.

Advantageously the stent further comprises a valvular structure having a plurality of leaflets, preferably two to six leaflets that are mounted onto a self-expanding stent frame. The valvular structure is arranged in the inferior section and is attached to the stent frame at at least some of the eyelets of the inferior section. In this way a well-functioning replacement valve is provided.

It is conceivable that the leaflets may be attached using means which may include, but are not limited to, sutures, glue, welds, clamps, barbs, hooks and other miscellaneous methods of attachment at the commissures and along the belly of the leaflets.

By attaching the leaflets and/or the valvular structure at the eyelets, the eyelets not only impart flexibility to the stent frame, but also provide an easy to use attachment point for the valvular structure.

The material of the valvular structure can be made from fixed allograft or xenograft pericardial tissue, polymeric or fabric material and can be made from one continuous piece of material or from several different pieces of material that are possibly also made of different kinds of material. The pieces may be attached to each other using methods including, but not limited to, glue, epoxy, staples or suture.

It is preferred if an anti-coagulant, anti-thrombogenic, or anti-platelet is added in an active or passive manner to the pericardium comprising the leaflets and/or cuff material.

In this connection it should be noted that an anti-coagulant or anti-thrombogenic, or anti-platelet can advantageously also be added in an active or passive manner to the stent frame or to all other components of the implant and stent respectively.

The valvular structure described may be continuous or mated to increase paravalvular sealing. The pericardium comprising the cuff material and/or the leaflets may be continuous or mated to increase fluid washout of the leaflets or other features to decrease clot formation. Having regard to the specific design of the leaflets, this may have a rounded or square belly shape.

Preferably an external diameter of the superior section is larger than an external diameter of the inferior section.

By providing a stent where the superior section has an increased outer diameter relative to the outer diameter of the inferior section, each section of the stent has a primary function. the primary function of the superior section is then the attachment of the stent to the patients' anatomy by means of an interference fit, whereas the primary function of the inferior section is then the accommodation and correct functioning of the valve within the stent in order to obtain an implant that not only functions correctly but also prevents paravalvular leakage in the best possible way.

Advantageously the inferior section has an at least substantially cylindrical shape with an outer surface of the cylindrical shape having a convex outer surface.

In particular an apex of the convex outer surface is designed to provide an interference fit to anchor the valve to the native, calcified aortic annulus and to prevent paravalvular leakage in this part of the blood vessel.

Advantageously the superior section has an outer shape that tapers outwardly towards a distal end of the stent from the inferior section. Preferably the outer shape of the superior section resembles the bowl of a champagne glass coupe. In this way a best possible attachment of the superior section of the stent to the patients' anatomy is established by means of an interference fit.

Advantageously the stent further comprises attachment means that are different from the plurality of eyelets and that are optionally provided with a radiopaque marker.

By providing at least one second kind of attachment means, such as tabs or paddles, at the stent, the attachment of the stent to a delivery system can be facilitated, so that the implant comprising the stent can be transported within the patients' body using e.g. minimally invasive techniques. For this purpose the attachment means may include passive or active mechanisms to attach to corresponding mating features in the delivery system. It is conceivable that either mechanism also uses electrical, mechanical or magnetic components for securement of the prosthesis within the delivery system.

Alternatively or additionally the attachment means can be provided to further aid the attachment of the implant comprising the stent to a blood vessel to avoid migration of the implant in time.

Advantageously the attachment means include radiopaque markers that aid the tracking of the implant during the transport of the delivery system within the patients' body. The radiopaque markers provided at the attachment means can in this respect be configured like the radiopaque markers provided at the eyelets.

Preferably the stent is configured to be deployed by means of a catheter having a French size between 14 and 24 Fr (14,67 mm and 25,13 mm).

Using a catheter of such small size reduces vascular trauma during implantation, thus making the implantation of such a stent less traumatic to a patients' body.

The use of smaller sized catheters is achievable due to the increase in flexibility of the stent that allows a stent to be crimped into a smaller catheter.

Preferably the stent comprises a material selected from the group of materials comprising superelastic material, shape memory material and combinations of said materials. Such materials provide the stent with further flexibility and hence aid the implantation of the stent.

In a further aspect the present invention relates to a use of a stent as described herein for orthotopic and heterotoptic applications at locations in the proximity of the atrioventricular valves.

In a further aspect the present invention relates to a method of delivering and deploying an implant comprising a stent in accordance with at least one of the preceding claims and a surgical valve, the method comprising the steps of:
- inserting the implant into a human or animal body;
- placing the implant in the vicinity of the aortic annulus;
- expanding the stent in the vicinity of the aortic annulus; and
- attaching the implant to a blood vessel by means of at least one of an interference fit, sutures, staples, adhesively bonding, clamps and clips.

By means of this method the implant can advantageously be deployed using minimally invasive techniques at the correct location of deployment.

During a transcatheter procedure, a catheter is inserted at a minimally invasive access route. Typical examples include femoral, aortic, and apical access sites. Prior to the procedure, the implant is loaded onto a small catheter, typically 24Fr (8mm diameter) or smaller. Once loaded, the catheter is guided to and deployed at the site of the native valve under fluoroscopy guidance. Deployment and placement of the implant can be achieved through balloon expansion or the use of a self-expanding valve platform.

The advantages described in connection with the stent correspond to those or are similar to those that can be obtained by means of the method in accordance with the invention.

The invention will be described in detail by means of embodiments and with reference to the drawings. These show preferred deployment locations and embodiments of the stent. The features described in the dependent claims, in the description and in the drawings may be configured in various combinations, which are encompassed by this document. The drawings show:
- Fig. 1: a side view of a bare stent;
- Fig. 2: a view of an implant comprising a stent, a valvular apparatus and cuff material;
- Fig. 2A: a detailed view of eyelets of a stent;
- Fig. 2B: a further detailed view of eyelets of a stent, with struts of the stent comprising sutures;
- Fig. 2C: a further detailed view of eyelets of a stent, with the eyelets being provided with radiopaque markers
- Fig. 3: an implant deployed at an annular position of the aortic valve;
- Fig. 4: an implant in a delivery system traversing an arch of a blood vessel;
- Fig. 5: a view of a partially deployed stent; and
- Fig. 6: a bare stent having attachment tabs.

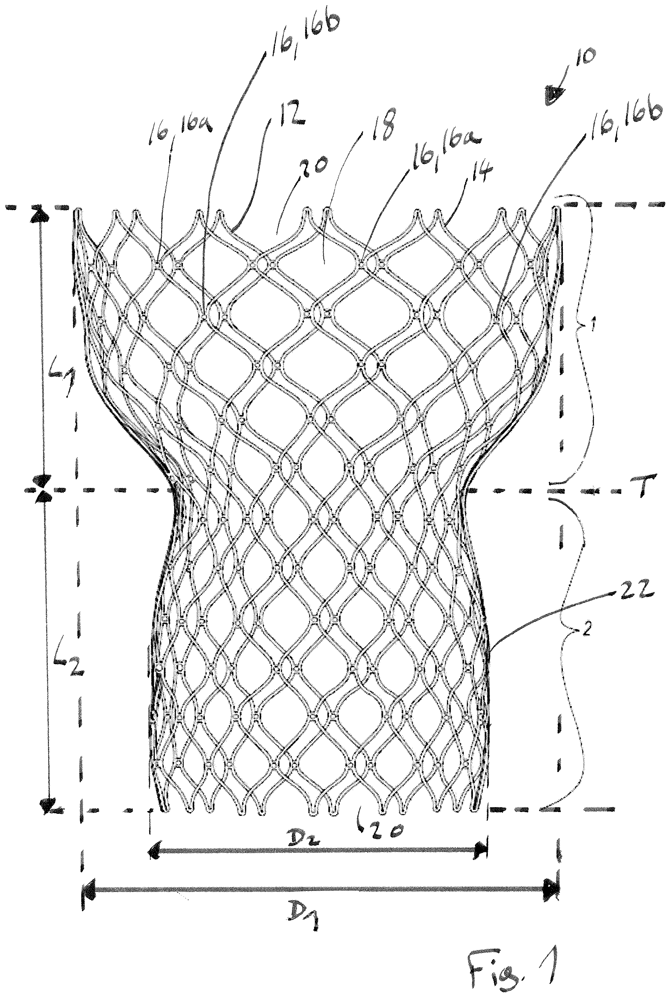

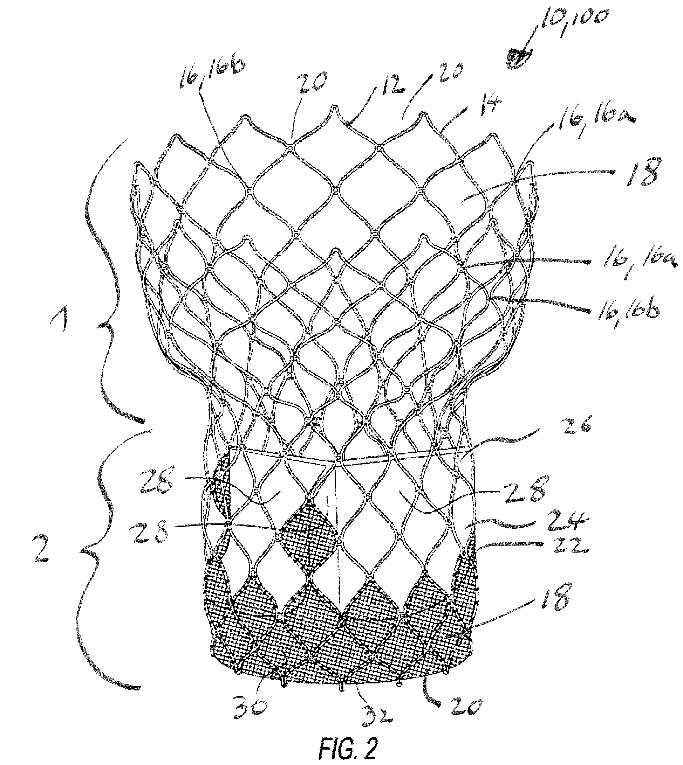

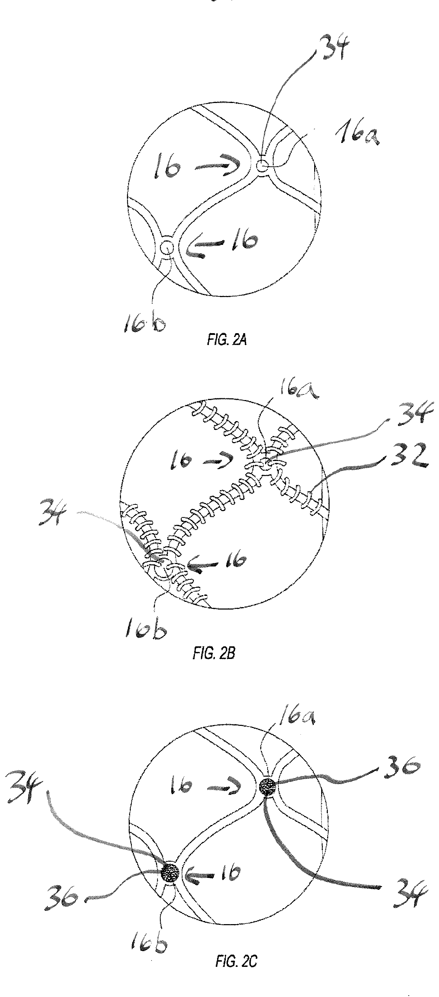

Fig. 1 shows a side view of a stent 10 of an implant 100 (see Fig. 2). The stent 10 is shown in an expanded state also known as the deployed state. The stent 10 comprises a self-expandable stent frame 12 formed from a plurality of elongate endless struts 14 that are connected one to another at a plurality of points of connection 16a. The points of connection 16a of the endless struts 14 are each formed by means of an eyelet 16.

Each individual endless strut 14 also has several points of constriction 16b when viewed along its length. The points of constriction are likewise each formed by an eyelet 16. Thus, once an endless strut 14 has been constricted at certain points along its length each strut has an undulating shape with two end points and several eyelets 16 combining the endless struts 14 at points of constriction 16b.

The eyelets 16 forming the points of constriction 16b and the points of connection 16a of the plurality of elongate endless struts 14 thus attach at each of the endless struts 14 to form a plurality of stent cells 18.

The stent frame 12 thus has a web-like structure, with each individual stent cell 18 of the web-like structure having an approximately curved diamond shape. At each of its ends the stent further comprises open stent cells 20.

Each stent cell 18 in the present example if formed of four sides and four vertices. The four sides of the stent cells 18 are respectively formed by sections of the struts 14 and the vertices are either formed by an end of a strut 14 or an eyelet 16.

The stent 10 has two distinct sections a superior section 1 and an inferior section 2. It should be noted in this connection that when reference is made to the term inferior in this application, then that part of the implant 100 respectively of the stent 10 is currently being referred to that is situated nearest the soles of the feet of a patient (not shown) in relation to a specific reference point having regard to the position of placement of the stent 10; inferior in this regard is the opposite of superior. Superior consequently refers to that part of the stent 10 that is situated farthest away from the soles of the feet of a patient in relation to the specific reference point.

An outer contour of the stent 10 of Fig. 1 can be described as having the following shapes, an approximately cylindrical shape in the inferior section 2 that has a slightly convex outer surface 22, the inferior section 2 transitions into the superior section 2 at a transition T that corresponds to a mathematical turning point, with the superior section 1 then tapering again outwardly from the transition T towards a distal end of the stent 10. At its most distal end the stent 10 may be selected to taper slightly inwardly, outwardly or to continue in an at least substantially straight way.

Generally speaking the outer contour is selected to adapt to the shape of the blood vessel into which it is fitted with the superior section 1 being adapted to create a tight interference fit with the blood vessel to ensure that the positioning of the stent 10 does not vary in time.

A maximum outer diameter D₁ of the superior section 1 is larger than a maximum outer Diameter D₂ of the inferior section 2. Preferably the maximum outer Diameter D₂ of the inferior section 2 amounts to between 40 and 90% of the maximum outer diameter D₁ of the superior section 1. Preferably the expanded external diameter D₁ of the superior section may be between 16mm and 50mm.

A length L₁ of the superior section 1 is shorter than a length L₂ of the inferior section 2. Preferably the length L₁ amounts to between 50 to 95% of the length L₂.

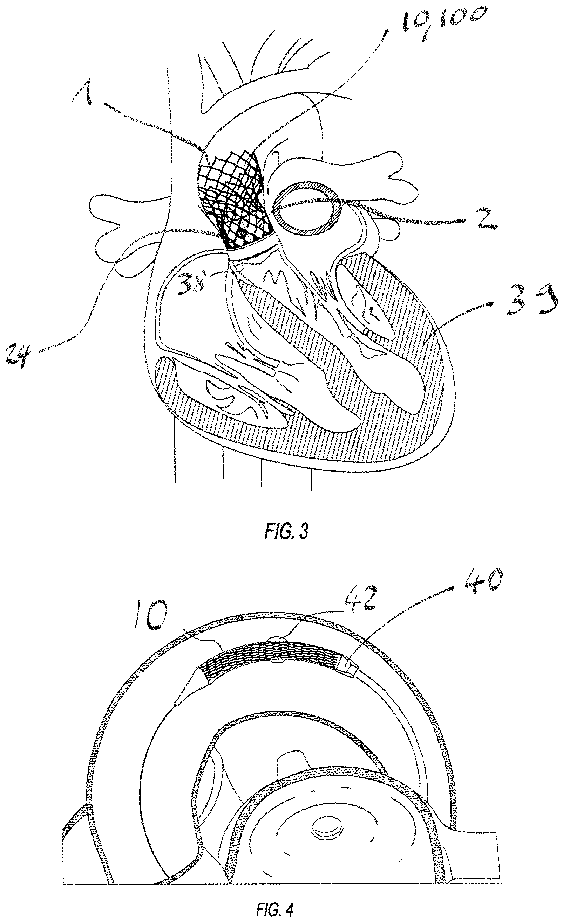

The superior section 1 is designed to have an interference fit with the aorta superior to the aortic annulus (see Fig. 3). This interference fit may add minor, but additional, stability to the valve once positioned.

Due to the fact that the stent cells 18 are larger in the superior section 1 than in the inferior section 2, the superior section 1 is more flexible than the inferior section 2. The size of the stent cells 18 is determined by the spacing between eyelets 16. The spacing between eyelets 16 is larger in the superior section 1 than in the inferior section 2.

Fig. 2 shows an expanded implant 100 including the stent 10 and a valvular structure 24 comprising a valve 26 having a plurality of leaflets 28 and cuff material 30 arranged in the inferior section 2. The cuff material 30 extends from the bottom of the stent 10 up to approximately the middle of the inferior section 2. The valvular structure is attached to the stent frame 12 via a plurality of sutures 32.

The material of the cuff material 30 and of the leaflets 28 may be fixed pericardial tissue. For each of the two sections 1, 2, it is conceivable that the radial strength of the stent 10 is tailored specifically for anchoring and valve performance. The eyelets 16 are designed to enhance the manufacturability of the implant via aiding the attachment of the cuff material 30 or leaflets 28 to the stent frame 12.

The stent 10 shown in Figs. 1 and 2 does not comprise any eyelets at its inferior and superior ends. The vertex of the outermost stent cells 18 is formed by the strut 14 itself in each case.

Nevertheless it is conceivable that the ends could include an eyelet, so that the struts respectively start and end at an eyelet (not shown). Likewise it is possible that the struts have an end which does not end in a vertex of a stent cell (also not shown).

The Figs. 2A to 2C show various detailed views of eyelets 16 respectively forming a point of connection 16a and a point of constriction 16b.

Fig. 2A shows two eyelets 16 of a bare stent frame 12. The aperture 34 of the eyelets 16 has a circular shape. It should be noted that the circular shape of the aperture 34 is only a design option and could also be selected oval or elliptical etc. The eyelets 16 are designed to enhance the flexibility of the stent 10, thereby they optimize the ability of the implant to traverse a blood vessel into which it is to be placed.

The diameter of the eyelets 16 and its attachment to the stent struts 14 may also be varied to increase the flexibility of the stent cells 18 relative to each other. The eyelets 16 can be circular, elliptical or rectangular in geometry. The dimensions of the eyelets 16 may be varied with respect to the dimensions of the stent cells 18 and struts 14 to control flexibility across each row of stent cells 18.

As shown in Fig. 2B the plurality of eyelets 16 is also formed to enhance the attachment of further components such as the cuff material 30 and the respective leaflets 28 of the valve 26, with the attachment being provided in the form of sutures 32 in this example.

Fig. 2C shows how the eyelets 16 may be used to enhance a visualization of the stent 10 in a patient through the addition of radiopaque markers 36 within the aperture 34 of the eyelets 16. The radiopaque markers 36 can be attached via swaging, luer attachment, crimping, welding or other similar methods. The radiopaque marker 36 may consist of, but is not limited to, tantalum, gold, palladium, platinum, platinum-iridium, and other radiopaque materials or combinations thereof.

Figs. 2A to 2C respectively show eyelets 16 that each form a respective origin of the points of connection 16a and of the points of constriction 16b at the corners of the respective stent cell 18 between respective sections of the elongate struts 14.

The stent 10 described herein is for a valve prosthesis that is suitable for the transcatheter replacement of the aortic valve to treat patients with aortic stenosis. Using transcatheter techniques, the implant will be placed either directly at the site of the aortic annulus 38 (see Fig. 3). It is conceivable that the device may be implanted using transcatheter techniques through incisions in a femoral artery, heart apex, aortic artery, or similar locations to access the desired implantation site. For a transcatheter placement of the device, a delivery system 40 (see Fig. 4) may be used to deliver the implant through the vasculature to the desired implantation site (aortic annulus). If used in multiple valvular positions in the heart, it is conceivable that multiple stents 10 may be delivered via a singular delivery system.

The stent 10 respectively the implant 100 shown in Fig. 3 is positioned in the vicinity of the aortic valve in order to regulate the flow of blood out of the heart during systole. Fig. 3 shows the placement of the deployed implant 100 at an annular position to restore native function and positioning. In this position the bottom end of the inferior section 2 of the valve is aligned with the native annular plane.

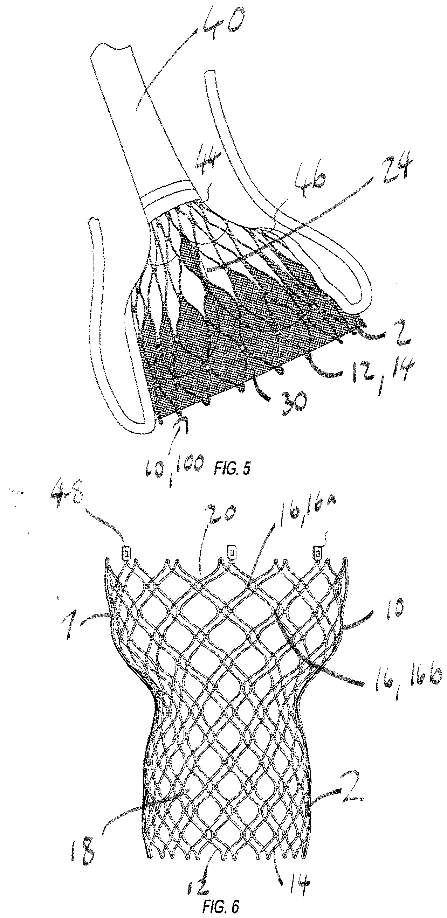

Once the stent 10 has been placed at its intended target position at or near the site of the diseased native aortic annulus it is released from the delivery mechanism 40 (see Fig. 5) and is expanded into the deployed state from the un-deployed state. For this reason the implant prosthesis is a self-expandable stent 10. This means in a non-deployed state it is not expanded whereas in a deployed state the stent 10 is expanded.

It is also possible to deploy the implant 100 into the ascending aorta and hence superior to the native aortic annulus (not shown). Likewise it is possible to deploy the implant at a subannular location (not shown), no lower than the height of the cuff to maintain annular apposition, in this way the bottom of the inferior terminus is lined up below the native annular plane. In all of these positions the inferior section is arranged proximal to the heart 39, so that a blood flowing through the stent 10 flows from the heart first through the inferior section 2 and then through the superior section 1 of the stent 10.

The targeted landing zone for the valvular apparatus 24 of the stent 10 near the aortic annulus 38 varies due to use. To restore aortic function, it is conceivable that implantation may occur inferior to the native aortic annulus to a height not exceeding the shortest part of the cuff material 30. Further, if cuff material 30 height is greater than 10mm, protrusion into the annulus cannot be greater than 10mm so as to not interfere with heart conduction. It is also conceivable that implantation within the native aortic annulus 38 is favorable to restore heart function. To aid hemodynamic performance within the aorta or mitigate paravalvular leakage, it is conceivable that the implant may be placed superior to the native aortic valve 38, within the ascending aorta, distal to the head vessels. It is reasonable that the overall geometry of the stent 10 (height, diameter, dimensions of each region) are tailored to fit specific anatomical needs in patients.

It should be noted in this connection that when reference is made to the term proximal in this application, then that part of the implant 100 respectively of the stent 10 which is intended to be positioned closest to the heart 39 is currently being referred to. Consequently the term distal refers to that part of the implant 100 respectively of the stent 10 that is to be disposed remote from the heart 39.

Fig. 4 shows a crimped valve in a delivery system 40 traversing an arched blood vessel. The stent 10 is designed with regions 42 in which with the eyelets 16 are concentrated to enhance bending and the capability of steering the un-deployed stent 10 through blood vessels. In this way the placement of the stent 10 is less traumatic for a patient.

Fig. 5 shows a partially deployed stent 10. The eyelets 16 may be concentrated at planes in the stent 10 which will enable it to more fully deploy during the early stages of release from the delivery system 40. These may include placement of the eyelets 16 along a plane 46 within the valvular apparatus 24 attachment region or within a plane 44 superior of the valvular apparatus 24 attachment region.

During the release from the delivery system 40 the self-expandable stent 10 bit by bit expands from the un-deployed state into the deployed state and then presses against the blood vessel and adheres there by at least an interference fit. In the present example half of the inferior section 1 has been released and is in the deployed state, the remaining part of the stent 10 is still to be released from the delivery system 40.

Fig. 6 shows a bare stent having attachment means 48 in the form of tabs. These attachment means 48 enable the stent 10 to be attached to the delivery system 40 and allow the stent 10 to mate temporarily with the delivery system 40 for implant loading and deployment.

Once the stent 10 has been deployed and the delivery system 40 is completely removed the stent 10 can attached to the blood vessel 10 by means of sutures 32 that engage the stent 10 at at least some of the attachment means 48 and/or at at least some of the eyelets 16 in addition to the attachment by means of an interference fit.

It is conceivable that the stent 10 may be implanted by means of surgical methods. This includes direct implantation in the aortic annulus during an open heart procedure with coronary bypass. It is also conceivable that the stent 10 will be implanted in any combination or any single location of the four cardiac valve annuli.

It is also conceivable that the stent 10 is used for the treatment of aortic regurgitation. In such cases, it is reasonable that the placement of the valve is achieved either by surgery or transcatheter options. It is also conceivable that for the transcatheter options, the access sites may include but are not limited to the femoral artery, heart apex, aortic artery or similar locations to access the desired implant site.

It is also conceivable, but not relating to a stent falling under the scope of the claims, that the stent 10 is used as a heterotopic treatment for tricuspid or mitral regurgitation, being implanted in the inferior vena cava and/or the superior vena cava or the pulmonary arteries. Specifically, it is conceivable that the device is used at heterotopic locations to mitigate atrioventricular regurgitation.

### List of reference numerals:

- 1: distal section
- 2: proximal section
- 10: stent
- 12: stent frame
- 14: endless strut
- 16: eyelet
- 16a: point of connection
- 16b: point of constriction
- 18: stent cell
- 20: half cell
- 22: convex outer surface
- 24: valvular structure
- 26: valve
- 28: leaflet
- 30: cuff material
- 32: suture
- 34: aperture
- 36: radiopaque marker
- 38: aortic annulus
- 39: heart
- 40: delivery system
- 42: regions of concentrated eyelets
- 44: plane
- 46: plane
- 48: attachment means
- 100: implant

- D₁: diameter
- D₂: diameter
- L₁: length
- L₂: length
- T: transition

## Claims

1. A stent (10) for placement at the aortic annulus that is expandable from an un-deployed state into a deployed state and that comprises:
a stent frame (12) that has an inferior section (2) and a superior section (1) and that is formed from a plurality of elongate struts (14), with the plurality of elongate struts (14) being connected one to another at points of connection to form a plurality of stent cells (18) formed of vertices (16a, 16b) at the points of connection and respective sections of the plurality of elongate struts (14); and
a plurality of eyelets (16), with at least some of the plurality of eyelets (16) forming at least some of the vertices (16a, 16b) at the points of connection between the elongate struts (14)
**characterized in that**
at least some of the elongate struts (14) are endless struts (14) having an undulating shape with two end points and several eyelets (16), with the vertices of at least some of the stent cells (18) of the endless struts (14) respectively being formed by points of connection (16a) between adjacent elongate struts (14) and by points of constriction (16b) by means of which the endless struts (14) are respectively combined along their length to form stent cells (18) within an individual endless strut (14), and
**in that** at least some of the stent cells (18) have all of their vertices (16a, 16b) formed by the eyelets (16), with the eyelets (16) connecting respective sections of the elongate struts (14) such that the stent frame (12) has a web-like structure, with each individual stent cell (18) of the web-like structure having a curved diamond shape formed by said struts (14) and four vertices (16a, 16b).

2. A stent (10) in accordance with claim 1,
wherein at least some of the stent cells (18) consist of vertices (16a, 16b) formed by some of the plurality of eyelets (16) and of respective sections of the plurality of elongate struts (14).

3. A stent (10) in accordance with claim 1 or claim 2,
wherein all of the vertices (16a, 16b) of stent cells (18) formed in a common plane (44, 46) of the stent frame (12) are formed by some of the plurality of eyelets (16).

4. A stent (10) in accordance with any one of the preceding claims,
wherein at least some of the stent cells (18) only comprise at most three vertices (16a, 16b) formed by some of the plurality of eyelets (16); and/or wherein the vertices (16a, 16b) form corners of the plurality of stent cells (18).

5. A stent (10) in accordance with any one of the preceding claims,
wherein all of the points of constriction (16b) and/or all of the points of connection (16a) are formed by the plurality of eyelets (16).

6. A stent (10) in accordance with any one of the preceding claims,
wherein a respective end of an endless strut (14) does not comprise a point of connection (16a) or a point of constriction (16b); and/or wherein the plurality of eyelets (16) each form a respective origin of the points of connection (16a) and/or of the points of constriction (16b).

7. A stent (10) in accordance with any one of the preceding claims,
wherein the inferior section (2) comprises a larger number of eyelets (16) than the superior section (1).

8. A stent in accordance with any one of the preceding claims,
wherein the inferior section (1) is less flexible than the superior section (2).

9. A stent (10) in accordance with any one of the preceding claims,
wherein at least one of the plurality of eyelets (16) forming one of the vertices is provided with a radiopaque marker (36).

10. A stent (10) in accordance with any one of the preceding claims,
further comprising a cuff material (30), with the cuff material (30) being arranged to cover at least part of an inner and/or an outer surface of the stent frame (12) and/or with the cuff material (30) being arranged to at least substantially completely cover at least a part of the inferior section (2) and/or of the superior section (1).

11. A stent (10) in accordance with any one of the preceding claims,
further comprising a valvular structure (24) having a plurality of leaflets (28) that is arranged in the inferior section (2) and is attached to the stent frame (12) at at least some of the eyelets (16) of the inferior section (2).

12. A stent (10) in accordance with any one of the preceding claims,
wherein an external diameter of the superior section (1) is larger than an external diameter of the inferior section (2).

13. A stent (10) in accordance with any one of the preceding claims,
wherein the inferior section (2) has an at least substantially cylindrical shape with an outer surface of the cylindrical shape having a convex outer surface; and/or wherein the superior section (1) has an outer shape that tapers outwardly towards a distal end of the stent (10) from the inferior section (2).

14. A stent (10) in accordance with any one of the preceding claims,
further comprising attachment means (48) that are different from the plurality of eyelets (16) and that are optionally provided with a radiopaque marker; and/or wherein the stent (10) is configured to be deployed by means of a catheter having a French size between 14 and 24 Fr; Fr (14,67 mm and 25,13 mm); and/or wherein the stent comprises a material selected from the group of materials comprising superelastic material, shape memory material and combinations of said materials.

15. A stent (10) in accordance with any one of the preceding claims, wherein the stent frame (12) is laser cut from a tube of material and processed to form a continuous frame.

## Patentansprüche

1. Stent (10) zur Platzierung am Aortenanulus, der von einem nicht entfalteten Zustand in einen entfalteten Zustand expandierbar ist und der umfasst:
einen Stentrahmen (12), der einen unteren Abschnitt (2) und einen oberen Abschnitt (1) aufweist und der aus einer Vielzahl von länglichen Streben (14) gebildet ist, wobei die Vielzahl von länglichen Streben (14) an Verbindungspunkten miteinander verbunden sind, um eine Vielzahl von Stentzellen (18) zu bilden, die aus Scheitelpunkten (16a, 16b) an den Verbindungspunkten und jeweiligen Abschnitten der Vielzahl von länglichen Streben (14) gebildet sind; und
eine Vielzahl von Ösen (16), wobei mindestens einige der Vielzahl von Ösen (16) mindestens einige der Scheitelpunkte (16a, 16b) an den Verbindungspunkten zwischen den länglichen Streben (14) bilden,
**dadurch gekennzeichnet, dass**
mindestens einige der länglichen Streben (14) endlose Streben (14) sind,
die eine Wellenform mit zwei Endpunkten und einer Vielzahl von Ösen (16) aufweisen, wobei die Scheitelpunkte mindestens einiger der Stentzellen (18) der endlosen Streben (14) jeweils durch Verbindungspunkte (16a) zwischen benachbarten länglichen Streben (14) und durch Verengungspunkte (16b) gebildet sind, mittels derer die endlosen Streben (14) jeweils entlang ihrer Länge kombiniert sind, um Stentzellen (18) innerhalb einer einzelnen endlosen Strebe (14) zu bilden, und
dass alle Scheitelpunkte (16a, 16b) mindestens einiger der Stentzellen (18) durch die Ösen (16) gebildet sind, wobei die Ösen (16) jeweilige Abschnitte der länglichen Streben (14) derart verbinden, dass der Stentrahmen (12) eine netzartige Struktur aufweist, wobei jede einzelne Stentzelle (18) der netzartigen Struktur eine gekrümmte Rautenform aufweist, die durch die Streben (14) und vier Scheitelpunkte (16a, 16b) gebildet ist.

2. Stent (10) nach Anspruch 1,
wobei mindestens einige der Stentzellen (18) aus Scheitelpunkten (16a, 16b) bestehen, die durch einige der Vielzahl von Ösen (16) und jeweilige Abschnitte der Vielzahl von länglichen Streben (14) gebildet sind.

3. Stent (10) nach einem der Ansprüche 1 oder 2,
wobei alle Scheitelpunkte (16a, 16b) von Stentzellen (18), die in einer gemeinsamen Ebene (44, 46) des Stentrahmens (12) gebildet sind, durch einige der Vielzahl von Ösen (16) gebildet sind.

4. Stent (10) nach einem der vorhergehenden Ansprüche,
wobei mindestens einige der Stentzellen (18) nur höchstens drei Scheitelpunkte (16a, 16b) umfassen, die durch einige der Vielzahl von Ösen (16) gebildet sind; und/oder wobei die Scheitelpunkte (16a, 16b) Ecken der Vielzahl von Stentzellen (18) bilden.

5. Stent (10) nach einem der vorhergehenden Ansprüche,
wobei alle der Verengungspunkte (16b) und/oder alle der Verbindungspunkte (16a) durch die Vielzahl von Ösen (16) gebildet sind.

6. Stent (10) nach einem der vorhergehenden Ansprüche,
wobei ein jeweiliges Ende einer endlosen Strebe (14) keinen Verbindungspunkt (16a) oder keinen Verengungspunkt (16b) umfasst; und/oder wobei die Vielzahl von Ösen (16) jeweils einen jeweiligen Ursprung der Verbindungspunkte (16a) und/oder der Verengungspunkte (16b) bildet.

7. Stent (10) nach einem der vorhergehenden Ansprüche,
wobei der untere Abschnitt (2) eine größere Anzahl von Ösen (16) als der obere Abschnitt (1) umfasst.

8. Stent nach einem der vorhergehenden Ansprüche,
wobei der untere Abschnitt (1) weniger flexibel als der obere Abschnitt (2) ist.

9. Stent (10) nach einem der vorhergehenden Ansprüche,
wobei mindestens eine der Vielzahl von Ösen (16), die einen der Scheitelpunkte bildet, mit einem röntgendichten Marker (36) versehen ist.

10. Stent (10) nach einem der vorhergehenden Ansprüche,
ferner umfassend ein Manschettenmaterial (30), wobei das Manschettenmaterial (30) angeordnet ist, um mindestens einen Teil einer inneren und/oder einer äußeren Oberfläche des Stentrahmens (12) zu bedecken, und/oder wobei das Manschettenmaterial (30) angeordnet ist, um mindestens einen Teil des unteren Abschnitts (2) und/oder des oberen Abschnitts (1) mindestens im Wesentlichen vollständig zu bedecken.

11. Stent (10) nach einem der vorhergehenden Ansprüche,
ferner umfassend eine Klappenstruktur (24) mit einer Vielzahl von Segeln (28), die in dem unteren Abschnitt (2) angeordnet ist und an dem Stentrahmen (12) an mindestens einigen der Ösen (16) des unteren Abschnitts (2) befestigt ist.

12. Stent (10) nach einem der vorhergehenden Ansprüche,
wobei ein Außendurchmesser des oberen Abschnitts (1) größer als ein Außendurchmesser des unteren Abschnitts (2) ist.

13. Stent (10) nach einem der vorhergehenden Ansprüche,
wobei der untere Abschnitt (2) eine mindestens im Wesentlichen zylindrische Form aufweist, wobei eine äußere Oberfläche der zylindrischen Form eine konvexe äußere Oberfläche aufweist; und/oder wobei der obere Abschnitt (1) eine äußere Form aufweist, die sich nach außen zu einem distalen Ende des Stents (10) von dem unteren Abschnitt (2) verjüngt.

14. Stent (10) nach einem der vorhergehenden Ansprüche,
ferner umfassend ein Befestigungsmittel (48), das sich von der Vielzahl von Ösen (16) unterscheidet und das optional mit einem röntgendichten Marker versehen ist; und/oder wobei der Stent (10) konfiguriert ist, um mittels eines Katheters mit einer French-Größe zwischen 14 und 24 Fr (14,67 mm und 25,13 mm) entfaltet zu werden, und/oder wobei der Stent ein Material umfasst, das aus der Gruppe von Materialien ausgewählt ist, die superelastisches Material, Formgedächtnismaterial und Kombinationen der Materialien umfasst.

15. Stent (10) nach einem der vorhergehenden Ansprüche, wobei der Stentrahmen (12) aus einem Rohr aus Material lasergeschnitten und verarbeitet wird, um einen kontinuierlichen Rahmen zu bilden.

## Revendications

1. Stent (10) destiné à être placé au niveau de l'anneau aortique, qui peut être mis en extension depuis un état non-déployé jusque dans un état déployé, et qui comprend :
un châssis de stent (12) qui a une section inférieure (2) et une section supérieure (1) et qui est formé à partir d'une pluralité de tiges allongées (14), la pluralité de tiges allongées (14) étant connectées les unes aux autres au niveau de points de connexion pour former une pluralité de cellules de stent (18) formées de sommets (16a, 16b) au niveau des points de connexion et de sections respectives de la pluralité de tiges allongées (14) ; et
une pluralité d'œillets (16), certains au moins de la pluralité d'œillets (16) formant certains au moins des sommets (16a, 16b) au niveau des points de connexion entre les tiges allongées (14),
**caractérisé en ce que**
certaines au moins des tiges allongées (14) sont des tiges sans fin (14) ayant une forme ondulante avec deux points d'extrémité et plusieurs œillets (16), les sommets de certaines au moins des cellules de stent (18) des tiges sans fin (14) étant respectivement formés par des point de connexion (16a) entre des tiges allongées adjacentes (14) et par des point de constriction (16b) au moyen desquels les tiges sans fin (14) sont respectivement combinées le long de leur longueur pour former des cellules de stent (18) à l'intérieur d'une tige sans fin individuelle (14), et
**en ce que** certaines au moins des cellules de stent (18) ont la totalité de leurs sommets (16a, 16b) formés par les œillets (16), les œillets (16) connectant des sections respectives des tiges allongées (14) de telle sorte que le châssis de stent (12) a une structure analogue à une toile, chaque cellule de stent individuelle (18) de la structure analogue à une toile ayant une forme de losange incurvé formé par lesdites tiges (14) et quatre sommets (16a, 16b).

2. Stent (10) selon la revendication 1,
dans lequel certaines au moins des cellules de stent (18) sont constitués de sommets (16a, 16b) formés par certains de la pluralité d'œillets (16), et de sections respectives de la pluralité de tiges allongées (14).

3. Stent (10) selon la revendication 1 ou 2,
dans lequel la totalité des sommets (16a, 16b) de cellules de stent (18) formées dans un plan commun (44, 46) du châssis de stent (12) sont formés par certains de la pluralité d'œillets (16).

4. Stent (10) selon l'une quelconque des revendications précédentes,
dans lequel certaines au moins des cellules de stent (18) comprennent uniquement au plus trois sommets (16a, 16b) formés par certains de la pluralité d'œillets (16) ; et/ou
dans lequel les sommets (16a, 16b) forment des coins de la pluralité de cellules de stent (18).

5. Stent (10) selon l'une quelconque des revendications précédentes,
dans lequel la totalité des points de constriction (16b) et/ou la totalité des points de connexion (16a) sont formés par la pluralité d'œillets (16).

6. Stent (10) selon l'une quelconque des revendications précédentes,
dans lequel une extrémité respective d'une tige sans fin (14) ne comprend pas de point de connexion (16a) ou de point de constriction (16b) ; et/ou
dans lequel la pluralité d'œillets (16) forment chacun une origine respective des points de connexion (16a) et/ou des points de constriction (16b).

7. Stent (10) selon l'une quelconque des revendications précédentes,
dans lequel la section inférieure (2) comprend un plus grand nombre d'œillets (16) que la section supérieure (1).

8. Stent (10) selon l'une quelconque des revendications précédentes,
dans lequel la section inférieure (2) est moins flexible que la section supérieure (1).

9. Stent (10) selon l'une quelconque des revendications précédentes,
dans lequel l'un au moins de la pluralité d'œillets (16) formant l'un des sommets est doté d'un marqueur radio-opaque (36).

10. Stent (10) selon l'une quelconque des revendications précédentes,
comprenant en outre un matériau de manchon (30), le matériau de manchon (30) étant agencé pour recouvrir une partie au moins d'une surface intérieure et/ou extérieure du châssis de stent (12) et/ou le matériau de manchon (30) étant agencé pour recouvrir au moins sensiblement complètement une partie au moins de la section inférieure (2) et/ou de la section supérieure (1).

11. Stent (10) selon l'une quelconque des revendications précédentes,
comprenant en outre une structure valvulaire (24) ayant une pluralité de lames (28) qui est agencée dans la section inférieure (2) et qui est attachée au châssis de stent (12) au niveau de certains au moins des œillets (16) de la section inférieure (2).

12. Stent (10) selon l'une quelconque des revendications précédentes,
dans lequel un diamètre externe de la section supérieure (1) est plus grand qu'un diamètre externe de la section inférieure (2).

13. Stent (10) selon l'une quelconque des revendications précédentes,
dans lequel la section inférieure (2) a une forme au moins sensiblement cylindrique, une surface extérieure de la forme cylindrique ayant une surface extérieure convexe ; et/ou
dans lequel la section supérieure (1) a une forme extérieure qui est effilée vers l'extérieur en direction d'une extrémité distale du stent (10) depuis la section inférieure (2).

14. Stent (10) selon l'une quelconque des revendications précédentes,
comprenant en outre un moyen d'attache (48) qui est différent de la pluralité d'œillets (16) et qui est en option doté d'un marqueur radio-opaque ; et/ou
dans lequel le stent (10) est configuré pour être déployé au moyen d'un cathéter ayant une taille en Charrière entre 14 et 24 Ch ; Ch (14,67 mm et 25,13 mm) ; et/ou
dans lequel le stent comprend un matériau sélectionné parmi le groupe de matériaux constitué d'une matériau super-élastique, d'une matériau à mémoire de forme, et de combinaisons desdits matériaux.

15. Stent (10) selon l'une quelconque des revendications précédentes,
dans lequel le châssis de stent (12) est découpé au laser à partir d'un tube de matériau et est traité pour former un châssis continu.
